# EUROPÄISCHE PATENTSCHRIFT

(11) **EP 3 264 077 B1**
(45) Veröffentlichungstag und Bekanntmachung des Hinweises auf die Patenterteilung: **03.07.2019**
(21) Anmeldenummer: 17000547.4
(22) Anmeldetag: 03.04.2017
(51) Int. Cl.: G01N 31/00, G01N 33/18

(54) **VERFAHREN ZUR BESTIMUNG DES TOC IN EINER FLÜSSIGEN PROBE UND BESTIMMUNG DES TN UND/ODER DES TP**
METHOD FOR DETERMINING THE TOC IN A LIQUID SAMPLE AND DETERMINING THE TN AND/OR THE TP
PROCÉDÉ DE DÉTERMINATION DU TOC DANS UN ÉCHANTILLON LIQUIDE ET DÉTERMINATION DU TN ET/OU DU TP

(30) Priorität: 10.05.2016 DE 102016005604
(43) Veröffentlichungstag der Anmeldung: 03.01.2018
(73) Patentinhaber: Elementar Analysensysteme GmbH, 63505 Langenselbold (DE)
(72) Erfinder: Löser, Danny, D-60285 Frankfurt (DE); Sieper, Hans-Peter, D-63571 Geinhausen (DE)
(74) Vertreter: Grimm, Ekkehard

(56) Entgegenhaltungen:
- CN-A- 103 159 284
- CN-A- 104 764 740
- DE-A1- 10 058 046
- JP-A- H09 127 005
- JP-A- 2001 056 332
- JP-A- 2006 075 731
- US-A1- 2016 018 376

## Beschreibung

Die vorliegende Erfindung betrifft ein Verfahren zur Bestimmung des TOC in einer flüssigen Probe und Bestimmung des TN und/oder des TP.

Die Bestimmung des TOC (gesamten organischen Kohlenstoffs) in einer flüssigen Probe ist allgemein bekannt und wird in allen Bereichen vorgenommen, wo der gesamte organische Kohlenstoff bestimmt werden soll, um daraus beispielsweise Verunreinigungen von Wasser ableiten zu können. Solche Bereiche sind insbesondere die Trinkwasser-, Oberflächenwasser- und Abwasseranalytik.

Bei den bekannten Verfahren zur Bestimmung des TOC wird die flüssige Probe in einer kleinen Menge in ein beheiztes Aufschlussgefäß eingegeben und ein Trägergas, beispielsweise Stickstoff oder synthetische Luft, in das Aufschlussgefäß eingeleitet. Anschließend wird ein Reagenz, eine saure Peroxodisulfat-Lösung (Na₂S₂O₈-Lsg), zugegeben und das Aufschlussgefäß wird mit UV-Licht bestrahlt, um dadurch eine Oxidation des gesamten organischen Kohlenstoffs in der Probe zu CO₂ zu erreichen. Danach wird das in der flüssigen Probe gebildete CO₂ mit einem Infrarot-Detektor bestimmt.

In sehr vielen Fällen ist es erwünscht, wenn nicht erforderlich, dass von einer flüssigen Probe neben dem TOC auch Informationen über den darin vorhandenen TN (Total Nitrogen - gesamten Stickstoff) und/oder den TP (Total Phosphor - gesamten Phosphor) erhalten werden. Mit bekannten Verfahren ist es nur möglich, den TP in der flüssigen Probe und den TN in der flüssigen Probe jeweils in einer speziell für die jeweilige Analyse vorgesehene Analysevorrichtung zu bestimmen. Dies hat auch zur Folge, dass für jeden Analysevorgang eine erneute Probenmenge der flüssigen Probe benötigt wird, die jeweils in ein Aufschlussgefäß eingegeben werden muss.

Die JP H09 127005 A betrifft ein Verfahren für die Langzeitbestimmung eines Bestandteils, wie beispielweise TN, TP und TOC, in Wasser. Eine Wasserprobe wird in das Reaktionsrohr eines photochemischen Reaktionsteils eingegeben, der pH-Wert wird auf 11 eingestellt und TiO₂-Pulver wird als Katalysator hinzugefügt. Reine Luft wird in den unteren Teil des Reaktionsrohrs des photochemischen Reaktionsteils in eine Wasserprobe hinein geblasen und Schwarzlicht und eine Niederdruck-Quecksilberlampe werden eingeschaltet, eine Photooxidationsreaktion wird vorgenommen und die Wasserprobe wird filtriert und in eine Messzelle überführt.

Die CN 104 764 740 A betrifft eine Vorrichtung und ein Verfahren für eine kontinuierliche, simultane On-line-Messung des TOC/des Gesamtphosphors einer Probe.

Die DE 100 58 046 A1 beschreibt ein Verfahren zur Quantifizierung und chromatographischen Charakterisierung des organisch gebundenen Kohlenstoffs in Reinstwässern. Die dem organisch gebundenen Kohlenstoff zugrunde liegenden Stoffgruppen werden in der zu analysierenden Probe mittels eines flüssigkeitschromatographischen Trennverfahrens aufgetrennt. Der in der Probe vorhandene anorganisch gebundene Kohlenstoff wird nach Ansäuerung und unter Zugabe eines Trägergases in einem membranlosen Entgasungsmodul als Kohlendioxid ausgetrieben. Dann wird organisch gebundener Kohlenstoff oxidiert, der organisch gebundene Kohlenstoff wird dann nach Oxidation zu Kohlendioxid und Zugabe eines Trägergases als Kohlendioxid ausgetrieben. Das aus dem organisch gebundenen Kohlenstoff freigesetzte Kohlendioxid wird schließlich infrarotspektrometrisch quantifiziert.

Die JP 2001 056332 A beschreibt einen Wasser-Analysierer, der geeignet ist, TN, TP und organische Verunreinigungen zu messen.

Der vorliegenden Erfindung liegt die Aufgabe zugrunde, ein Verfahren anzugeben, mit dem neben der Bestimmung des TOC (gesamten organischen Kohlenstoffs) in einer flüssigen Probe auch eine Bestimmung des TN und/oder des TP aus einer flüssigen Probemenge vorgenommen werden kann.

Gelöst wird diese Aufgabe durch ein Verfahren mit den Merkmalen des Anspruchs 1. Vorteilhafte Ausgestaltungen des Verfahrens ergeben sich aus den abhängigen Ansprüchen.

Das erfindungsgemäße Verfahren zeichnet sich dadurch aus, dass die zu analysierende flüssige Probe nur einmal in einer kleinen, aber ausreichenden Menge (Milliliter sind ausreichend) in das eingesetzte Aufschlussgefäß eingegeben werden muss. Dies hat den Vorteil, dass für alle Analysevorgänge dieselbe Probe, ohne dass sie für jeden Analysevorgang erneut dem Aufschlussgefäß zugeführt werden muss, verwendet wird.

Gemäß dem erfindungsgemäßen Verfahren wird zunächst die flüssige Probe, die analysiert werden soll, bereitgestellt. Für einen automatisierten Ablauf kann dann, oder auch vor der Probeneingabe, eine Abfrage erfolgen, ob neben dem TOC auch der TN und/oder der TP dieser flüssigen Probe bestimmt werden soll. Falls die Abfrage ergibt, dass der TOC und der TN bestimmt werden sollen, wird das entsprechende Programm (Programm 120) gestartet. Es kann eine Aufforderung erfolgen, die flüssige Probe in das beheizte Aufschlussgefäß einzufüllen und Trägergas (Stickstoff oder synthetische Luft) in das beheizte Aufschlussgefäß einzuleiten. Dann wird ein Reagenz A, vorzugsweise eine saure Peroxodisulfat-Lösung (Na₂S₂O₈), definiert, das bedeutet eine zuvor bestimmte Dosiermenge, in das Aufschlussgefäß eingeleitet und die Probe im Aufschlussgefäß mit UV-Licht bestrahlt, um dadurch eine Oxidation der flüssigen Probe zu bewirken.

In einem ersten Aufschluss wird der organische Kohlenstoff (C) in der Probe zu CO₂ umgesetzt, der in C-N-Bindung vorliegende Stickstoff in Ammonium und andere Stickstoffverbindungen in Nitrat überführt. Dann wird der TOC mit einem IR-Detektor ermittelt

Anschließend erfolgt eine definierte Zugabe eines Reagenz B, vorzugsweise einer alkalischen Peroxodisulfat-Lösung, in das beheizte Aufschlussgefäß. Die Probe im Aufschlussgefäß wird erneut mit UV-Licht bestrahlt, und in einem zweiten Aufschluss wird durch Oxidation das gebildete Ammonium zu Nitrat umgesetzt. Die flüssige Probe wird dann entgast, so dass die Probe dadurch blasenfrei von dem Aufschlussgefäß in einen TN-Detektor geführt werden kann und der in der Probe vorhandene TN gemessen und bestimmt werden kann.

Das Peroxodisulfat wird durch UV-Strahlung vorzugsweise mit einer Wellenlänge von 254 nm und/oder 185 nm aktiviert, wobei als reaktive Spezies Sulfatradikale wirken. Die Bestrahlung mit beiden Wellenlängen stellt eine vollständige Oxidation selbst stabilster Verbindungen sicher.

Falls die anfängliche Abfrage ergibt, dass der TOC und der TP bestimmt werden sollen, wird der entsprechende Programmablauf (Programm 140) gestartet.

Nachdem die flüssige Probe in das beheizte Aufschlussgefäß eingeleitet ist, wird ein TFägergas (Stickstoff oder synthetische Luft) zugeführt sowie ein Reagenz C, bevorzugt eine Peroxodisulfat-Lösung (Na₂S₂O₈) und Schwefelsäure (H₂SO₄), in das Aufschlussgefäß eingegeben. Nun wird die Probe im Aufschlussgefäß mit UV-Licht bestrahlt und dadurch der organische Kohlenstoff (C) zu CO₂ und die Phosphor-/Phosphatverbindungen zu Orthophosphat umgesetzt. Das Gas wird dann in einen IR-Detektor zur Bestimmung des TOC eingeleitet. Für die Messung der TP wird die flüssige Probe entgast, so dass ein Teil der Probe blasenfrei von dem Aufschlussgefäß in einen TP-Detektor überführt werden kann, um den TP zu messen.

Falls die Abfrage ergibt, dass der TOC, der TN und der TP bestimmt werden sollen (Programm 160), wird die flüssige Probe in das beheizte Aufschlussgefäß eingegeben, ein Trägergas (Stickstoff oder synthetischer Luft) in das beheizte Aufschlussgefäß eingeleitet, ein Reagenz C, vorzugsweise Peroxodisulfat-Lösung (Na₂S₂O₈) und Schwefelsäure (H₂SO₄), in das Aufschlussgefäß hinzugegeben, und die Probe im Aufschlussgefäß mit UV-Licht bestrahlt.

In einem ersten Aufschluss wird nun der organische Kohlenstoff (C) zu CO₂ und die Phosphor-/Phosphatverbindungen in Orthophosphat umgewandelt. In der Probe vorliegende Stickstoffverbindungen werden zu Nitrat umgewandelt, wobei der in C-N-Bindung vorliegende Stickstoff in Ammonium überführt wird. Dann wird der TOC in der flüssigen Probe mit einem IR-Detektor bestimmt. Für die Messung des TP wird die flüssige Probe entgast, so dass ein Teil der Probe blasenfrei von dem Aufschlussgefäß in einen TP-Detektor überführt werden kann, um den TP zu messen.

Durch die Entgasung und dadurch blasenfreie Zuführung der Probe in den TP-Detektor ist gewährleistet, dass das Messsignal fehlerfrei aufgenommen werden kann.

Nun erfolgt eine definierte Zugabe eines Reagenz B, vorzugsweise einer alkalischen Peroxodisulfat-Lösung, in das beheizte Aufschlussgefäß und die Probe wird in dem Aufschlussgefäß mit UV-Licht bestrahlt, bis alle gebundenen Stickstoffverbindungen zu Nitrat oxidiert sind.

In einem zweiten Aufschluss wird durch Oxidation das gebildete Ammonium zu Nitrat umgesetzt. Es folgt eine Entgasung der Probe und dadurch eine blasenfreie Überführung der entgasten Probe in einen TN-Detektor, wo der Anteil an TN in der Probe gemessen wird.

Falls die Abfrage ergibt, dass nur der TOC bestimmt werden soll (Programm 180), wird die flüssige Probe in das beheizte Aufschlussgefäß eingegeben, ein Trägergas (Stickstoff oder synthetische Luft) in das Aufschlussgefäß eingeleitet, ein Reagenz A, vorzugsweise eine saure Peroxodisulfat-Lösung (Na₂S₂O₈), hinzugefügt und die Probe im Aufschlussgefäß mit UV-Licht bestrahlt. Organischer Kohlenstoff (C) in der Probe wird dadurch zu CO₂ umgesetzt und der in der Probe enthaltene TOC wird mit einem IR-Detektor bestimmt.

Falls bei einer anfänglichen Abfrage keine Eingabe durch einen Benutzer vorgenommen wurde, ein Eingabefehler festgestellt wurde oder kein Programm gestartet wurde, wird ein erneuter Durchlauf der jeweiligen Abfrage durchgeführt.

Ein vorteilhaftes Merkmal des erfindungsgemäßen Verfahrens, wie es vorstehend beschrieben ist, ist dadurch gegeben, dass für alle vorgesehenen Bestimmungen die flüssige Probe nur einmal in das Aufschlussgefäß eingegeben wird und der entsprechende Schritt der Eingabe der flüssigen Probe in das beheizte Aufschlussgefäß und die Einleitung von Stickstoff oder synthetischer Luft als Trägergas nur einmal durchgeführt wird. Für die jeweilige Messung wird, nachdem der dazu benötigte Teil der Probenmenge aus dem Aufschlussgefäß abgeführt ist, der Probenausgang ausgangsseitig des Aufschlussgefäßes über ein Absperrventil geschlossen und die vorgesehene Analyse durchgeführt. Nach Abschluss der Analyse wird das Absperrventil erneut geöffnet, um eine erneute Teilmenge der flüssigen Probe aus dem Aufschlussgefäß einer weiteren Analyse zuzuführen. Diese Abläufe werden wiederholt, bis alle vorgesehenen Analysen mit der sich im Aufschlussgefäß befindlichen Probe durchgeführt sind.

Das Verfahren zur Bestimmung des TOC (gesamten organischen Kohlenstoffs) und zur Bestimmung des TN (Total Nitrogen - gesamten Stickstoffs) in einer flüssigen Probe, wie es bereits vorstehend beschrieben wurde (Programm 120), stellt eine eigenständige Erfindung gemäß Anspruch 4 dar; in Bezug auf die hierzu durchzuführenden Verfahrensschritte wird auf die vorstehenden Erläuterungen verwiesen (Programm 120).

In den vorstehend angegebenen Verfahren wird vorzugsweise als Reagenz A eine saure Peroxodisulfat-Lösung (Na₂S₂O₈) verwendet. Für das Reagenz B wird zweckmäßigerweise eine alkalische Peroxodisulfat-Lösung eingesetzt. Als Reagenz C werden vorzugsweise eine Peroxodisulfat-Lösung (Na₂S₂O₈) und Schwefelsäure (H₂SO₄) verwendet.

Die Vorrichtung für die Durchführung der erfindungsgemäßen Verfahren, wie sie vorstehend beschrieben sind, um den TOC (gesamten organischen Kohlenstoffs) in einer flüssigen Probe und optional den TN (Total Nitrogen - gesamten Stickstoffs) und/oder den TP (Total Phosphor - gesamten Phosphors) zu bestimmen, und welche selbst nicht Teil der vorliegenden Erfindung ist, umfasst ein Aufschlussgefäß mit einem Proben-Aufnahmeraum zum Aufnehmen einer flüssigen Probenmenge. Das Aufschlussgefäß ist beheizbar, um die flüssige Probe zu erwärmen. Dem Proben-Aufnahmeraum ist eine UV-Lampe zugeordnet, so dass eine in den Proben-Aufnahmeraum eingegebene flüssige Probe mit UV-Licht bestrahlt werden kann, um die flüssige Probe zu oxidieren.

Das Aufschlussgefäß besitzt, in einer vertikalen Anordnung gesehen, an seinem oberen Ende eine Trägergas-Zuführung, um in den Proben-Aufnahmeraum ein Trägergas zuzuführen, das dazu dient, das Messgas zu transportieren und die Probe zu zirkulieren. Außerdem befindet sich an dem oberen Ende eine Proben-Zuführung für die flüssige Probe ebenso wie eine erste Reagenzien-Zuführung und eine zweiten Reagenzien-Zuführung sowie ein Messgasausgang für die TOC Messung. An dem gegenüberliegenden Ende, oder, in der vertikalen Anordnung gesehen, an seinem unteren Ende, befinden sich ein Proben-Ausgang. Über den Proben-Ausgang wird die für den jeweiligen Analysevorgang erforderliche Teilmenge der flüssigen Probe aus dem Aufschlussgefäß entnommen, während und über den Proben-Ausgang mit dem Ende der Analysevorgänge die nicht mehr benötigte flüssige Probe aus dem Aufschlussgefäß abgelassen wird.

Wesentlich ist auch, dass dem Proben-Aufnahmeraum eine Proben-Füllhöhe zugeordnet ist, bis zu der die flüssige Probe in den Proben-Aufnahmeraum eingefüllt wird. Die Trägergas-Zuführung ist so dimensioniert, dass zumindest deren Ende unterhalb der Proben-Füllhöhe endet, so dass dieses Ende in die flüssige Probe eintaucht. Auch ist vorgesehen, zumindest das Ende der Trägergas-Zuführung und das Ende der Proben-Zuführung als Kapillarrohr auszubilden. Gleiches gilt für zumindest das Ende der Reagenzien-Zuführung.

Ein besonders zweckmäßiger Aufbau ergibt sich durch ein Aufschlussgefäß, dessen Proben-Aufnahmeraum als Ringraum ausgebildet ist, der auf seiner Innenseite durch ein Innenrohr und der auf seiner Außenseite durch ein Außenrohr begrenzt ist. Bei diesem Aufbau ist die mindestens eine UV-Lampe in den Innenraum des Innenrohrs eingesetzt und erstreckt sich vorzugsweise über die gesamte Länge des Innenrohrs. Insbesondere bei diesem rohrförmigen Aufbau ist das Aufschlussgefäß mit der Rohrachse vertikal angeordnet.

An den Proben-Ausgang des Aufschlussgefäßes schließt sich, in Strömungsrichtung gesehen, eine Absperrung an, die dazu dient, die jeweils für einen Analysevorgang erforderliche Teilmenge der flüssigen Probe dem Aufschlussgefäß zu entnehmen. Dieser Absperrung folgt ein Drei/Zweiwegeventil, das in seiner einen Stellung die flüssige Probe durch eine Kühleinrichtung führt, während es in seiner anderen Stellung mit einen Abfallbehälter verbunden ist, um Reste aus dem Aufschlussgefäß zu entfernen.

Nach der Kühleinrichtung führt die flüssige Probe über eine Leitung zu einem Entgaser und zu einem Detektor. Der Entgaser besitzt einen Ausgang, als Kapillare ausgeführt, der mit der Atmosphäre verbunden ist; diese Kapillare, dient dazu, Luftblasen aus der definierten Probenmenge zu entfernen ohne viel Probenvolumen zu verbrauchen.

Durch die Entgasung kann die Probe von dem Aufschlussgefäß blasenfrei zu dem Detektor überführt werden.

Weitere Einzelheiten und Merkmale der Erfindung ergeben sich aus der nachfolgenden Beschreibung von Ausführungsbeispielen anhand der Zeichnung. In der Zeichnung zeigt
- Figur 1: ein Hauptprogramm 100 des erfindungsgemäßen Verfahrens,
- Figur 2: ein Unterprogramm 120, auf das in dem Hauptprogramm 100 der Figur 1 verwiesen ist, für die Bestimmung von TOC und TN,
- Figur 3: ein Unterprogramm 140, auf das in dem Hauptprogramm 100 der Figur 1 verwiesen ist, für die Bestimmung von TOC und TP,
- Figur 4: ein Unterprogramm 160, auf das in dem Hauptprogramm 100 der Figur 1 verwiesen ist, für die Bestimmung von TOC und TN und TP,
- Figur 5: ein Unterprogramm 180, auf das in dem Hauptprogramm 100 der Figur 1 verwiesen ist, für die Bestimmung von TOC,
- Figur 6: einen schematischen Aufbau einer beispielhaften Vorrichtung mit einem Aufschlussgefäß, und
- Figur 7: eine schematische, perspektivische Ansicht eines Aufschlussgefäßes, wie es in der Vorrichtung der Figur 6 verwendet ist

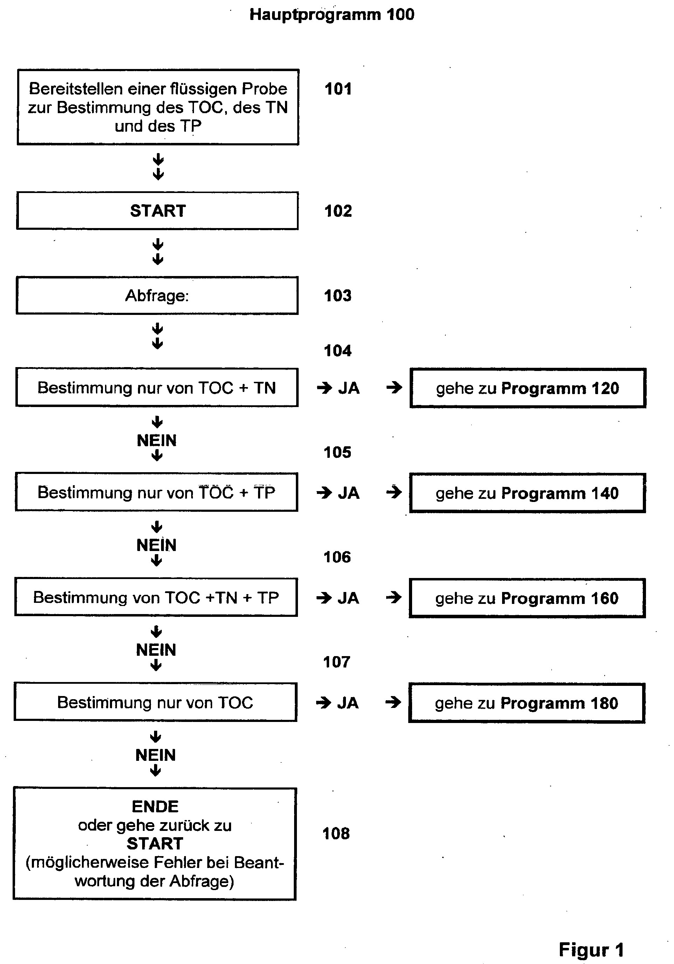

Das erfindungsgemäße Verfahren wird nachfolgend anhand der einzelnen Programme, wie sie in den Figuren 1 bis 5 dargestellt sind, erläutert.

Das Verfahren, um den TOC sowie den TN und/oder TP in einer flüssigen Probe zu bestimmen, kann in einer zumindest teilweise automatisierten Folge ablaufen, indem zunächst in einem Hauptprogramm 100, das in Figur 1 dargestellt ist, an den Anwender verschiedene Abfragen gerichtet werden. Es ist allerdings darauf hinzuweisen, dass ein solches Hauptprogramm entfallen kann, wenn der Anwender durch Vorgaben die von ihm gewünschten Analysevorgänge angibt, so dass unmittelbar das entsprechende Programm 120, 140, 160, oder 180 gestartet wird.

Wie das Diagramm des Hauptprogramms, das in Figur 1 dargestellt ist, zeigt, stellt der Anwender zunächst eine flüssige Probe bereit, für die der TOC, der TN und/oder der TP bestimmt werden sollen; Schritt 101.

Das angegebene Verfahren, und entsprechend die dazu vorgesehenen Programmabläufe, sehen vor, dass alle Bestimmungen, wie sie in dem Hauptprogramm 100 angegeben sind, in einem Analyse-Ablauf durchgeführt werden können, indem nur einmal eine Menge einer flüssigen Probe in ein Aufschlussgefäß eingegeben wird und von dieser Proben-Menge jeweils eine Teilmenge für jeden Bestimmungsvorgang verwendet wird.

Es folgt im Schritt 102 der START, gefolgt durch den Beginn verschiedener Abfragen im Schritt 103. Die nun folgenden Abfragen (Schritte 104 bis 107), die in der Reihenfolge geändert werden können, sind:

| | | |
|---|---|---|
| - Bestimmung nur von TOC + TN | Schritt 104 | gehe zu Programm 120 |
| - Bestimmung nur von TOC + TP | Schritt 105 | gehe zu Programm 140 |
| - Bestimmung von TOC +TN + TP | Schritt 106 | gehe zu Programm 160 |
| - Bestimmung nur von TOC | Schritt 107 | gehe zu Programm 180 |

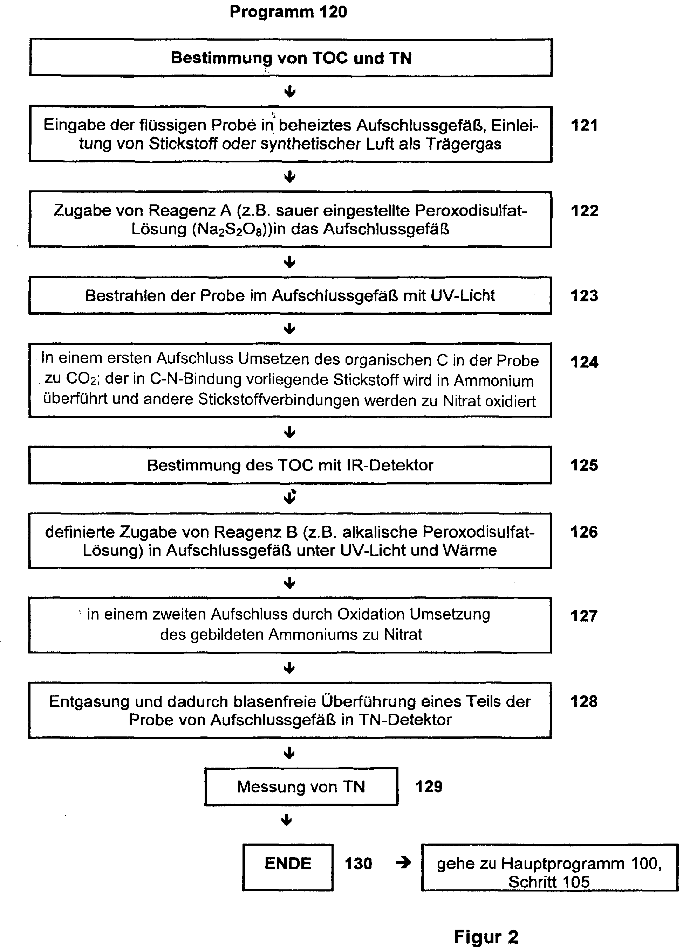

Wenn eine Abfrage positiv mit JA beantwortet wird, wird zu dem jeweils angegebenen Programm 120, 140, 160 und 180 der Figuren 2, 3, 4 und 5 übergegangen. Falls eine Abfrage mit NEIN beantwortet wird, geht der Ablauf zu der jeweils folgenden Abfrage weiter. Nachdem eines der Programme 120, 140, 160 und 180 mit der entsprechenden Bestimmung abgeschlossen ist, führt das entsprechende Programm zurück zu dem Hauptprogramm 100, um die nächste, noch nicht vorgenommene Abfrage für die nächste, folgende Bestimmung vorzunehmen.

Im Schritt 108 endet das Programm, oder, wenn alle Abfragen mit NEIN beantwortet wurden, wird nochmals zu Schritt 102 START zurückgegangen, da möglicherweise ein Fehler bei Beantwortung der Abfragen in den Schritten 104 bis 107 vorlag.

Falls Schritt 104 ergibt, dass TOC und TN bestimmt werden sollen, wird der Ablauf des Programms 120 (Figur 2) durchgeführt.

Es kann nochmals eine Aufforderung im Schritt 121 erfolgen, die flüssige Probe in das beheizte Aufschlussgefäß einzufüllen und Trägergas (Stickstoff oder synthetische Luft) in das beheizte Aufschlussgefäß einzuleiten. Dann wird im Schritt 122 ein Reagenz A, vorzugsweise eine saure Peroxodisulfat-Lösung (Na₂S₂O₈), in das Aufschlussgefäß eingeleitet und im Schritt 123 die Probe im Aufschlussgefäß mit UV-Licht bestrahlt.

In einem ersten Aufschluss, Schritt 124, wird der organische Kohlenstoff (C) in der Probe zu CO₂ umgesetzt und der in C-N-Bindung vorliegende Stickstoff wird in Ammonium und andere Stickstoffverbindungen in Nitrat überführt. Anschließend wird im Schritt 125 der TOC mit einem IR-Detektor ermittelt.

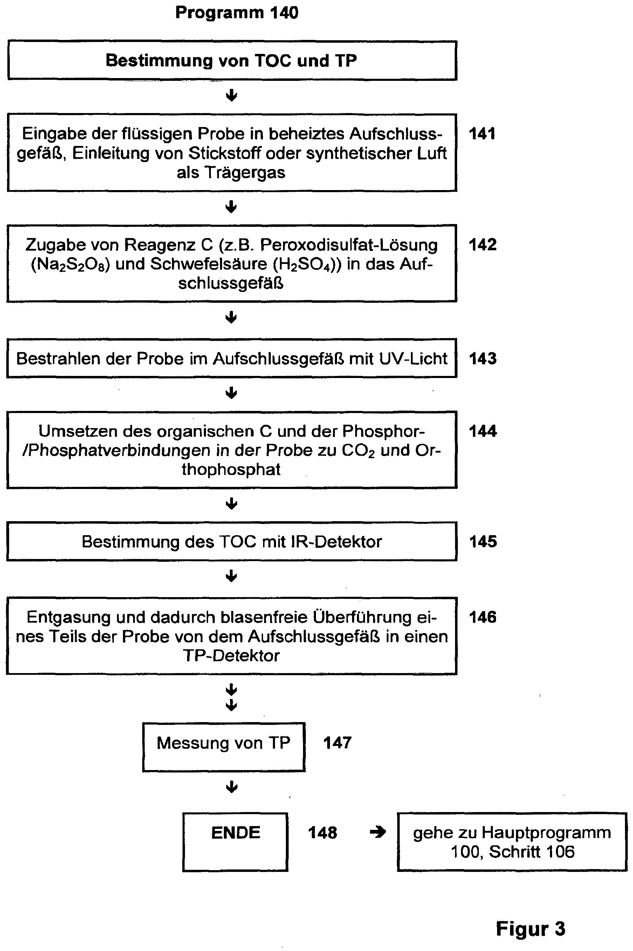

Nun folgt im Schritt 126 eine definierte Zugabe eines Reagenz B, vorzugsweise einer alkalischen Peroxodisulfat-Lösung, in das beheizte Aufschlussgefäß und die Probe im Aufschlussgefäß wird erneut mit UV-Licht bestrahlt. In einem zweiten Aufschluss (Schritt 127) wird durch Oxidation das gebildete Ammonium zu Nitrat umgesetzt. Im Schritt 128 wird die Probe dann entgast, so dass die Probe dadurch blasenfrei in einen TN-Detektor geführt wird, um den in der Probe vorhandene TN zu messen und zu bestimmen (Schritt 129). Im Schritt 130 endet das Programm 120, und es wird zu dem Hauptprogramm100, dort zu Schritt 105, zurückgegangen. Falls Schritt 104 ergibt, dass TOC und TP bestimmt werden sollen, wird der Ablauf des Programms 140 (Figur 3) durchgeführt.

Es kann nochmals eine Aufforderung im Schritt 141 erfolgen, die flüssige Probe in das beheizte Aufschlussgefäß einzufüllen und Trägergas (Stickstoff oder synthetische Luft) in das beheizte Aufschlussgefäß einzuleiten, falls dies der erste Analysenvorgang ist. Im Schritt 142 erfolgt die Zugabe eines Reagenz C, bevorzugt einer Peroxodisulfat-Lösung (Na₂S₂O₈) und Schwefelsäure (H₂SO₄), in das Aufschlussgefäß und eine Bestrahlung der Probe mit UV-Licht, Schritt 143, um dadurch den organischen Kohlenstoff (C) zu CO₂ und die Phosphor-/Phosphatverbindungen zu Orthophosphat umzusetzen (Schritt 144). Die Probe wird dann im Schritt145 einem IR-Detektor zur Bestimmung des TOC zugeführt. Dann wird im Schritt 146 für die Messung des TP die Probe entgast und blasenfrei in einen TP-Detektor überführt, um im Schritt 147 den TP zu messen.

Im Schritt 148 endet das Programm 140, und es wird zu dem Hauptprogramm 100, dort zu Schritt 106, zurückgegangen.

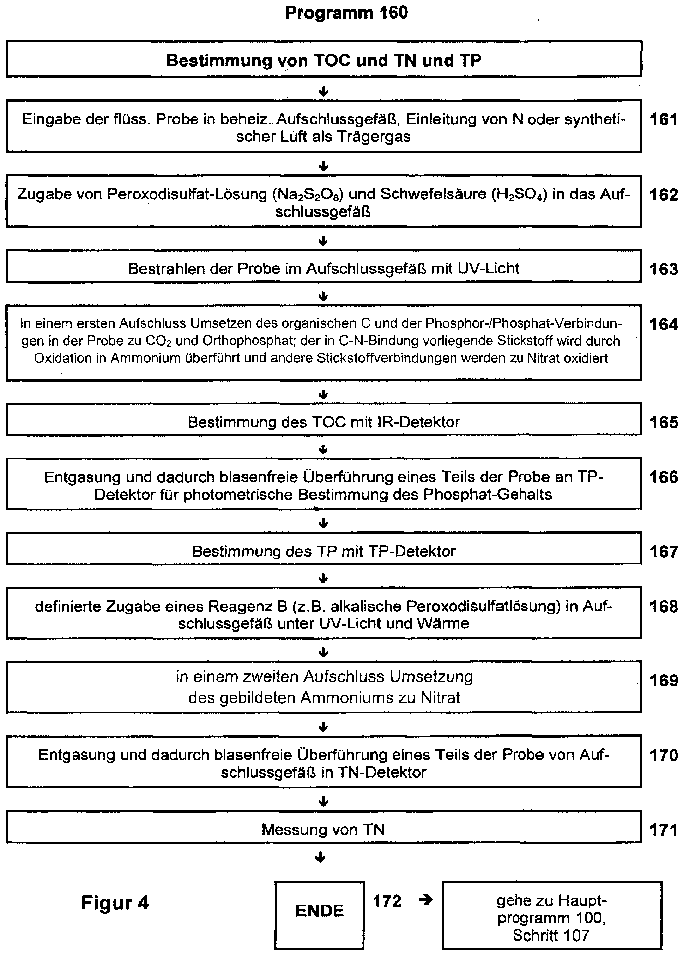

Falls Schritt 106 ergibt, dass TOC und TN und TP bestimmt werden sollen, wird der Ablauf des Programms 160 (Figur 4) durchgeführt.

Es kann nochmals eine Aufforderung im Schritt 161 erfolgen, die flüssige Probe in das beheizte Aufschlussgefäß einzufüllen und Trägergas (Stickstoff oder synthetische Luft) in das beheizte Aufschlussgefäß einzuleiten. Im Schritt 162 erfolgt die Zugabe eines Reagenz C, vorzugsweise Peroxodisulfat-Lösung (Na₂S₂O₈) und Schwefelsäure (H₂SO₄), in das Aufschlussgefäß und die Probe wird im Schritt 163 im Aufschlussgefäß mit UV-Licht bestrahlt.

Im Schritt 164 wird in einem ersten Aufschluss nun der organische Kohlenstoff (C) zu CO₂ und die Phosphor-/Phosphatverbindungen in Orthophosphat umgewandelt, wobei der in C-N-Bindung vorliegende Stickstoff in Ammonium und andere Stickstoffverbindungen in Nitrat überführt werden.

Dann wird im Schritt 165 der TOC in der Probe mit einem IR-Detektor bestimmt. Danach wird im Schritt 166 ein weiterer Teil der Probe dem Aufschlussgefäß entnommen und entgast, um diesen Teil der Probe blasenfrei in einen TP-Detektor zu überführen und den TP der Probe zu messen und zu bestimmen (Schritt 167). Durch die Entgasung ist eine blasenfreie Zuführung der Probe in den TP-Detektor gewährleistet.

Nun erfolgt im Schritt 168 eine definierte Zugabe eines Reagenz B, vorzugsweise einer alkalischen Peroxodisulfat-Lösung, in das beheizte Aufschlussgefäß und die Probe wird in dem Aufschlussgefäß mit UV-Licht bestrahlt.

In einem zweiten Aufschluss, Schritt 169, wird das gebildete Ammonium zu Nitrat umgesetzt. Es folgt im Schritt 170 eine Entgasung der Probe, so dass die für die Bestimmung dem Aufschlussgefäß entnommene Probenmenge blasenfrei in einem TN-Detektor überführt wird (Schritt 170), um im Schritt 171 den Anteil an TN in der Probe zu bestimmen. Im Schritt 172 endet das Programm 160 der Figur 4, und es wird zu dem Hauptprogramm100, dort zu Schritt 107, zurückgekehrt.

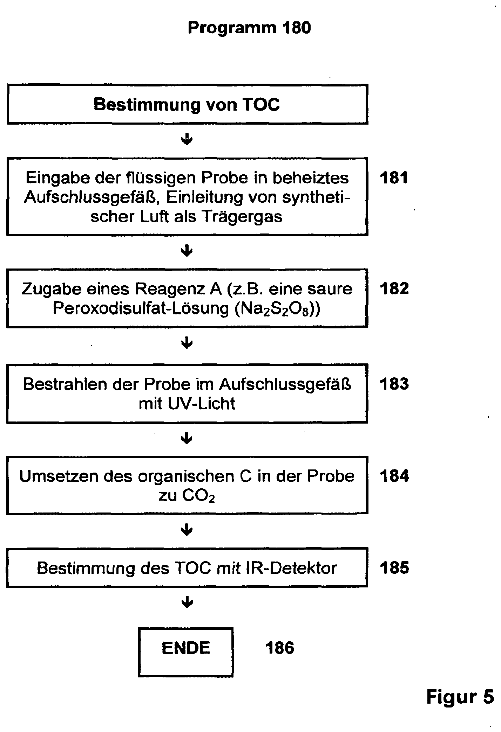

Falls Schritt 107 ergibt, dass nur der TOC der flüssigen Probe bestimmt werden soll, wird der Ablauf des Programms 180 (Figur 5) durchgeführt.

Es kann auch hier eine Aufforderung im Schritt 181 erfolgen, die flüssige Probe in das beheizte Aufschlussgefäß einzufüllen und Trägergas (Stickstoff oder synthetische Luft) in das beheizte Aufschlussgefäß einzuleiten. Im Schritt 182 erfolgt die Zugabe eines Reagenz A, vorzugsweise einer sauren Peroxodisulfat-Lösung (Na₂S₂O₈), und die Probe wird im Schritt 183 im Aufschlussgefäß mit UV-Licht bestrahlt. Im Schritt 184 wird organischer Kohlenstoff (C) in der Probe zu CO₂ umgesetzt. Dann wird, wie Schritt 185 zeigt, der in der Probe enthaltene TOC mit einem IR-Detektor bestimmt. Das Programm 180 endet schließlich mit Schritt 186.

Falls die einzelnen Abfragen in den Schritten 104 bis 107 nicht mit JA beantwortet werden, ist davon auszugehen, dass ein Eingabefehler vorliegt und kein Programm gestartet wurde. Deshalb wird das Programm im Schritt 108 zu Schritt 103 zurückgeführt, um die Abfragen der Schritte 104 bis 108 erneut zu durchlaufen.

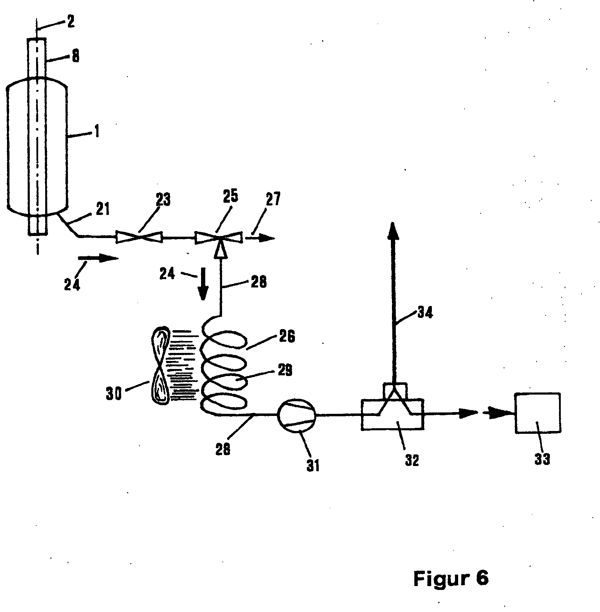

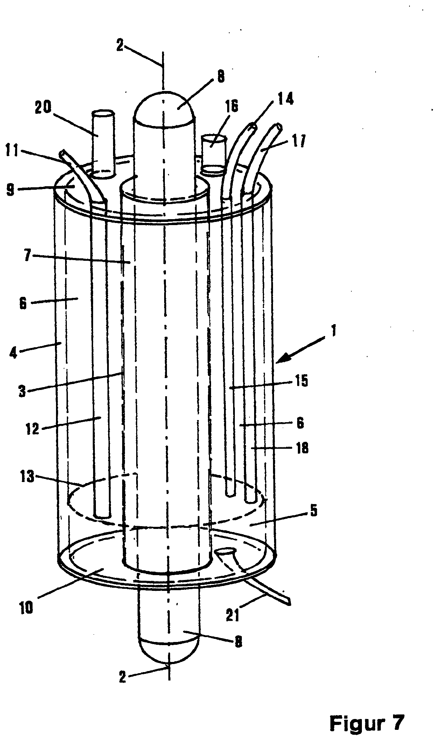

Die Vorrichtung, die zur Durchführung der einzelnen Verfahren (Programme), wie sie vorstehend beschrieben sind, eingesetzt wird, und die nicht Teil der Erfindung ist, ist schematisch in Figur 6 dargestellt, wobei das Aufschlussgefäß in einer vergrößerten detaillierten perspektiven Ansicht in Figur 7 gezeigt ist.

Wie die Figur 6 zeigt, umfasst die Vorrichtung, um die vorstehend beschriebenen Verfahren zur Bestimmung des TOC (gesamten organischen Kohlenstoffs) und des TN (Total Nitrogen - gesamter Stickstoff) und/oder des TP (Total Phosphor - gesamter Phosphor) in einer flüssigen Probe durchzuführen, ein Aufschlussgefäß 1, das detaillierter in Figur 7 dargestellt ist.

Wie die Figur 7 in einer schematischen Darstellung verdeutlicht, ist das Aufschlussgefäß 1 konzentrisch um eine Achse 2 aus im Wesentlichen einem Innenrohr 3 und aus einem mit Abstand dazu angeordneten Außenrohr 4, so dass zwischen dem Innenrohr 3 und dem Außenrohr 4 ein Proben-Aufnahmeraum 5 als Ringraum 6 ausgebildet ist, aufgebaut. In den durch das Innenrohr 3 begrenzten Innenraum 7 ist eine UV-Lampe 8 eingesetzt, mit der eine in den Proben-Aufnahmeraum 5 eingefüllte flüssige Probe mit UV-Licht bestrahlt werden kann.

Das Aufschlussgefäß 1, das mit seiner Achse 2 in einer vertikalen Anordnung ausgerichtet ist, ist auf der Oberseite mit einer oberen Abschlussplatte 9 und auf der Unterseite mit einer unteren Abschlussplatte 10 verschlossen. Das Außenrohr 4 bildet die äußere Hülle des Aufschlussgefäßes 1.

An der Oberseite des Aufschlussgefäßes 1 befindet sich im Bereich der oberen Aufschlussplatte 9 eine dem Proben-Aufnahmeraum 5 ein Trägergas über einen Gas-Eingang 11 zuführende Trägergas-Zuführung 12. Die Trägergas-Zuführung 12, als Röhrchen mit einem kleinen Innendurchmesser ausgebildet, reicht bis in den unteren Bereich des Proben-Aufnahmeraums 5 und endet dort unterhalb einer Proben-Füllhöhe, die durch eine unterbrochene Linie 13 angedeutet ist. Bis zu dieser Proben-Füllhöhe 13 wird über einen Proben-Eingang 14 einer als Röhrchen ausgebildeten Proben-Zuführung 15 die zu analysierende, flüssige Probe eingefüllt. Auch diese Proben-Zuführung 15 endet unterhalb der Proben-Füllhöhe 13.

Weiterhin befinden sich an dem oberen Ende des Aufschlussgefäßes 1 zwei Einfüllstutzen 16 und 17 für ein erstes Reagenz und ein zweites Reagenz, wobei eine Reagenz-Zuführung 17 mit einem Zuführrohr 18 verbunden ist, die sich bis in den unteren Bereich des Ringraums 6 bzw. des Proben-Aufnahmeraums 5 erstrecken, um je nach Erfordernis ein erstes Reagenz oder ein zweites Reagenz der flüssigen Probe zuzuführen. Auch befindet sich an dem oberen Ende des Aufschlussgefäßes 3 ein Gas-Ausgang 20, um den TOC zum IR-Detektor zu transportieren.

Im unteren Bereich des Aufschlussgefäßes 1 befinden sich, vorzugsweise in der unteren Abschlussplatte 10, ein Proben-Ausgang 21, der dazu dient, jeweils eine erforderliche Teilmenge der flüssigen Probe für die jeweilige Analyse abzuführen, und übrig gebliebene Probe in die Atmosphäre zu führen. Hierzu ist der Proben-Ausgang 21 mit einem nicht dargestellten Absperrventil versehen. Auch der Proben-Ausgang 21 kann über einen Absperrhahn oder ein Absperrventil 23 verschlossen oder geöffnet werden, das in Figur 6 gezeigt ist.

Vorzugsweise sind zumindest das Ende der Trägergas-Zuführung 12, zumindest das Ende der Proben-Zuführung 15 und zumindest das Ende des Zuführrohrs 18 für die Zuführung des jeweiligen Reagenz als Kapillarrohr ausgebildet.

Das Aufschlussgefäß 1 ist über eine nicht näher dargestellte Heizeinrichtung aufheizbar, um die in den Proben-Aufnahmeraum 5 eingefüllte flüssige Probe zu erwärmen, um dadurch den Oxidationsprozess zu beschleunigen.

Wie die Figur 6 zeigt, wird für den jeweiligen Analysevorgang, wie er vorstehend anhand der in den Figuren 2 bis 5 dargestellten Programme 120, 140, 160 und 180 beschrieben ist, eine Teilmenge der flüssigen Probe über das Öffnen des Absperrventils 23 entnommen. Diesem Absperrventil 23 folgt, in Strömungsrichtung der flüssigen Probe gesehen, angedeutet durch Strömungspfeile 24, ein Drei/Zweiwegeventil 25. Dieses Zweiwegeventil 25 führt die flüssige Probe in seiner einen Stellung zu einer Kühleinrichtung 26, während es in seiner anderen Stellung, angedeutet durch den Pfeil 27, mit Atmosphäre verbunden ist, um übrig gebliebene Probe zu entsorgen. Die Kühleinrichtung 26 besteht vorzugsweise aus einem spiralförmig verlaufenden Abschnitt 29, der die flüssige Probe transportierende Leitung 28, der durch ein Gebläse 30 mit Luft gekühlt wird. Diese Kühleinrichtung 26 dient dazu, die flüssige Probe abzukühlen.

Nach der Kühleinrichtung 26 wird der Transport der flüssigen Probe durch die Leitung 28 mittels einer Pumpe 31 unterstützt, die jedoch auch an einer anderen geeigneten Stelle in die Leitung 28 eingefügt werden kann.

Die flüssige Probe wird nach der Kühleinrichtung 26 über einen Entgaser 32 geführt, der Blasen aus der flüssigen Probe entfernt, so dass diese blasenfrei zu einem für den jeweiligen Analysevorgang erforderlichen Detektor, die in Figur 6 nur angedeutet und mit dem Bezugszeichen 33 bezeichnet sind, geführt wird.

Die Verwendung eines Entgasers 32 ist ein wesentliches Merkmal der Vorrichtung, da Blasen in der flüssigen Probe in dem jeweiligen Detektor 33 schlechte bzw. falsche oder keine Ergebnisse hervorrufen würden. Wie die Figur 6 zeigt, ist der Entgaser 32 über eine Kapillare 34 mit der Atmosphäre zur Abgabe der Luft verbunden, um Luftblasen entweichen zu lassen und dabei nicht zu viel der Probenmenge zu verlieren.

## Patentansprüche

1. Verfahren zur Bestimmung zumindest des TOC (gesamten organischen Kohlenstoffs) und optional Bestimmung des TN (Total Nitrogen - gesamten Stickstoffs) und/oder des TP (Total Phosphor - gesamten Phosphors) in einer flüssigen Probe mit folgenden Verfahrensschritten:
- Bereitstellen einer flüssigen Probe, die analysiert werden soll,
- Abfragen, ob neben dem TOC auch der TN und/oder der TP dieser flüssigen Probe bestimmt werden soll,
- falls die Abfrage ergibt, dass der TOC und der TN bestimmt werden sollen (Programm 120),
- Eingabe der flüssigen Probe in ein beheiztes Aufschlussgefäß,
- Einleitung von Stickstoff oder synthetischer Luft als Trägergas in das beheizte Aufschlussgefäß,
- Zugabe eines Reagenz A in das Aufschlussgefäß,
- Bestrahlen der Probe im Aufschlussgefäß mit UV-Licht,
- in einem ersten Aufschluss, Umsetzen des organischen C in der Probe zu CO₂, Umsetzen durch Oxidation der Stickstoffverbindungen zu Nitrat, wobei der in C-N-Bindung vorliegende Stickstoff in Ammonium überführt wird,
- Bestimmung des TOC mit einem IR-Detektor,
- definierte Zugabe eines Reagenz B in das beheizte Aufschlussgefäß,
- Bestrahlen der Probe im Aufschlussgefäß mit UV-Licht,
- in einem zweiten Aufschluss, Umsetzen durch Oxidation des gebildeten Ammoniums zu Nitrat,
- Entgasung und dadurch blasenfreie Überführung eines Teils der Probe von dem Aufschlussgefäß in einen TN-Detektor und
- Messung von TN,
- falls die Abfrage ergibt, dass der TOC und der TP bestimmt werden sollen (Programm 140),
- Eingabe der flüssigen Probe in ein beheiztes Aufschlussgefäß,
- Einleitung von Stickstoff oder synthetischer Luft als Trägergas in das Aufschlussgefäß,
- Zugabe eines Reagenz C in das Aufschlussgefäß,
- Bestrahlen der Probe im Aufschlussgefäß mit UV-Licht,
- Umsetzen des organischen C zu CO₂ und der Phosphor-/Phosphatverbindungen zu Orthophosphat in der Probe,
- Bestimmung des TOC mit einem IR-Detektor,
- Entgasung und dadurch blasenfreie Überführung eines Teils der Probe von dem Aufschlussgefäß in einen TP-Detektor,
- Messung von TP,
- falls die Abfrage ergibt, dass der TOC, der TN und der TP bestimmt werden sollen (Programm 160),
- Eingabe der flüssigen Probe in ein beheiztes Aufschlussgefäß,
- Einleitung von Stickstoff oder synthetischer Luft als Trägergas in das beheizte Aufschlussgefäß,
- Zugabe eines Reagenz C in das Aufschlussgefäß,
- Bestrahlen der Probe im Aufschlussgefäß mit UV-Licht,
- in einem ersten Aufschluss, Umsetzen des organischen C zu CO₂ und der Phosphor-/Phosphatverbindungen in Orthophosphat, Umsetzen der Stickstoffverbindungen zu Nitrat, wobei der in C-N-Bindung vorliegende Stickstoff in Ammonium überführt wird,
- Bestimmung des TOC mit einem IR-Detektor,
- Entgasung und dadurch blasenfreie Überführung eines Teils der Probe von dem Aufschlussgefäß in einen TP-Detektor,
- Messung von TP,
- definierte Zugabe eines Reagenz B in das beheizte Aufschlussgefäß,
- Bestrahlen der Probe im Aufschlussgefäß mit UV-Licht
- in einem zweiten Aufschluss, Umsetzen durch Oxidation des gebildeten Ammoniums zu Nitrat,
- Entgasung und dadurch blasenfreie Überführung eines Teils der Probe von dem Aufschlussgefäß in einen TN-Detektor,
- Messung von TN,
- falls die Abfrage ergibt, dass nur der TOC bestimmt werden soll (Programm 180),
- Eingabe der flüssigen Probe in ein beheiztes Aufschlussgefäß,
- Einleitung von Stickstoff oder synthetischer Luft als Trägergas in das Aufschlussgefäß,
- Zugabe eines Reagenz A in das Aufschlussgefäß,
- Bestrahlen der Probe im Aufschlussgefäß mit UV-Licht,
- Umsetzen des organischen C in der Probe zu CO₂ und
- Bestimmung des TOC mit einem IR-Detektor.

2. Verfahren nach Anspruch 1, **dadurch gekennzeichnet, dass** ein erneuter Durchlauf der jeweiligen Abfrage durchgeführt wird, um festzustellen, ob ein Eingabefehler auf die jeweilige Abfrage hin vorliegt, falls auf die jeweiligen Abfragen, ob neben dem TOC auch der TN und/oder der TP bestimmt werden soll, keine positive Antwort erfolgt ist.

3. Verfahren nach Anspruch 1 oder 2, **dadurch gekennzeichnet, dass** für alle vorgesehenen Bestimmungen die flüssige Probe nur einmal in das Aufschlussgefäß eingegeben wird und der entsprechende Schritt der Eingabe der flüssigen Probe in das beheizte Aufschlussgefäß und der Einleitung von Stickstoff oder synthetischer Luft als Trägergas nur einmal durchgeführt wird.

4. Verfahren zur Bestimmung des TOC (gesamten organischen Kohlenstoffs) und zur Bestimmung des TN (Total Nitrogen - gesamten Stickstoffs) in einer flüssigen Probe, **gekennzeichnet durch** folgende Verfahrensschritte (Programm 120):
- Eingabe der flüssigen Probe in ein beheiztes Aufschlussgefäß,
- Einleitung von Stickstoff oder synthetischer Luft als Trägergas in das beheizte Aufschlussgefäß,
- Zugabe eines Reagenz A in das Aufschlussgefäß,
- Bestrahlen der Probe im Aufschlussgefäß mit UV-Licht,
- in einem ersten Aufschluss, Umsetzen des organischen C in der Probe zu CO₂, Umsetzen der Stickstoffverbindungen zu Nitrat, wobei der in C-N-Bindung vorliegende Stickstoff zu Ammonium überführt wird,
- Bestimmung des TOC mit einem IR-Detektor,
- definierte Zugabe eines Reagenz B in das beheizte Aufschlussgefäß,
- Bestrahlen der Probe im Aufschlussgefäß mit UV-Licht,
- in einem zweiten Aufschluss, Umsetzen durch Oxidation des gebildeten Ammoniums zu Nitrat,
- Entgasung und dadurch blasenfreie Überführung eines Teils der Probe von dem Aufschlussgefäß in einen TN-Detektor und
- Messung von TN.

5. Verfahren nach einem der Ansprüche 1 bis 4, **dadurch gekennzeichnet, dass** das Reagenz A eine saure Peroxodisulfat-Lösung (Na₂S₂O₈) ist.

6. Verfahren nach einem der Ansprüche 1 bis 5, **dadurch gekennzeichnet, dass** das Reagenz B eine alkalische Peroxodisulfat-Lösung ist.

7. Verfahren nach einem der Ansprüche 1 bis 3, 5 oder 6, **dadurch gekennzeichnet, dass** das Reagenz C eine Peroxodisulfat-Lösung (Na₂S₂O₈) und Schwefelsäure (H₂SO₄) ist.

## Claims

1. Method for determining at least the TOC (total organic carbon) and optionally determining the TN (total nitrogen) and/or the TP (total phosphorus) in a liquid sample, comprising the following method steps:
- providing a liquid sample to be analyzed,
- querying whether the TN and/or TP of this liquid sample is to be determined in addition to the TOC,
- if the query shows that the TOC and the TN are to be determined (program 120),
- feeding the liquid sample into a heated decomposition vessel,
- introducing nitrogen or synthetic air as a carrier gas into the heated decomposition vessel,
- adding a reagent A to the decomposition vessel,
- irradiating the sample in the decomposition vessel with UV light,
- in a first decomposition, converting the organic C in the sample to CO₂, converting by oxidation of the nitrogen compounds to nitrate, wherein the nitrogen present in C-N bond is converted to ammonium,
- determining the TOC with an IR detector,
- defined addition of a reagent B to the heated decomposition vessel,
- irradiating the sample in the decomposition vessel with UV light,
- in a second decomposition, converting by oxidation of the formed ammonium to nitrate,
- degassing and thereby bubble-free transfer of part of the sample from the decomposition vessel into a TN detector, and
- measuring TN,
- if the query shows that the TOC and TP are to be determined (program 140),
- feeding the liquid sample into a heated decomposition vessel,
- introducing nitrogen or synthetic air as a carrier gas into the decomposition vessel,
- adding a reagent C to the decomposition vessel,
- irradiating the sample in the decomposition vessel with UV light,
- converting the organic C to CO₂ and the phosphorus/phosphate compounds to orthophosphate in the sample,
- determining the TOC with an IR detector,
- degassing and thereby bubble-free transfer of part of the sample from the decomposition vessel into a TP detector,
- measuring TP,
- if the query shows that the TOC, the TN and the TP are to be determined (program 160),
- feeding the liquid sample into a heated decomposition vessel,
- introducing nitrogen or synthetic air as a carrier gas into the heated decomposition vessel,
- adding a reagent C to the decomposition vessel,
- irradiating the sample in the decomposition vessel with UV light,
- in a first decomposition, converting the organic C to CO₂ and the phosphorus/phosphate compounds to orthophosphate, converting the nitrogen compounds to nitrate, wherein the nitrogen present in C-N bond is converted to ammonium,
- determining the TOC with an IR detector,
- degassing and thereby bubble-free transfer of part of the sample from the decomposition vessel into a TP detector,
- measuring TP,
- defined addition of a reagent B to the heated decomposition vessel,
- irradiating the sample in the decomposition vessel with UV light,
- in a second decomposition, converting by oxidation of the formed ammonium to nitrate,
- degassing and thus bubble-free transfer of part of the sample from the decomposition vessel to a TN detector,
- measuring TN,
- if the query shows that only the TOC is to be determined (program 180),
- feeding the liquid sample into a heated decomposition vessel,
- introducing nitrogen or synthetic air as a carrier gas into the decomposition vessel,
- adding a reagent A to the decomposition vessel,
- irradiating the sample in the decomposition vessel with UV light,
- converting the organic C in the sample to CO₂ and
- determining the TOC with an IR detector.

2. Method according to claim 1, **characterized in that** a new run of the respective query is performed in order to determine whether there is an input error in response to the respective query if no positive answer has been given to the respective queries as to whether, in addition to the TOC, the TN and/or the TP is also to be determined.

3. Method according to claim 1 or 2, **characterized in that** for all the intended determinations the liquid sample is fed into the decomposition vessel only once and the corresponding step of feeding the liquid sample into the heated decomposition vessel and introducing nitrogen or synthetic air as a carrier gas is carried out only once.

4. Method for determining the TOC (total organic carbon) and for determining the TN (total nitrogen) in a liquid sample, **characterized by** the following method steps (program 120):
- feeding the liquid sample into a heated decomposition vessel,
- introducing nitrogen or synthetic air as a carrier gas into the heated decomposition vessel,
- adding a reagent A to the decomposition vessel,
- irradiating the sample in the decomposition vessel with UV light,
- in a first decomposition, converting the organic C in the sample to CO₂, converting the nitrogen compounds to nitrate, wherein the nitrogen present in C-N bond is converted to ammonium,
- determining the TOC with an IR detector,
- defined addition of a reagent B to the heated decomposition vessel,
- irradiating the sample in the decomposition vessel with UV light,
- in a second decomposition, converting by oxidation of the formed ammonium to nitrate,
- degassing and thereby bubble-free transfer of part of the sample from the decomposition vessel into a TN detector, and
- measuring TN.

5. Method according to any of claims 1 to 4, **characterized in that** the reagent A is an acidic peroxodisulfate solution (Na₂S₂O₈).

6. Method according to any of claims 1 to 5, **characterized in that** the reagent B is an alkaline peroxodisulfate solution.

7. Method according to any of claims 1 to 3, 5 or 6, **characterized in that** the reagent C is a peroxodisulfate solution (Na₂S₂O₈) and sulfuric acid (H₂SO₄).

## Revendications

1. Procédé de détermination au moins du TOC (carbone organique total) et éventuellement de détermination du TN (azote total) et/ou du TP (phosphore total) dans un échantillon liquide, comprenant les étapes de procédé suivantes :
- mise à disposition d'un échantillon liquide qui doit être analysé,
- interrogation pour savoir si, en plus du TOC, le TN et/ou le TP de cet échantillon liquide doivent être également déterminés,
- au cas où l'interrogation a pour résultat que le TOC et le TN doivent être déterminés (programme 120),
- insertion de l'échantillon liquide dans un récipient d'attaque chauffé,
- introduction d'azote ou d'air synthétique à titre de gaz support dans le récipient d'attaque chauffé,
- addition d'un réactif A dans le récipient d'attaque,
- irradiation de l'échantillon présent dans le récipient d'attaque avec la lumière UV,
- au cours d'une première réaction d'attaque, conversion du C organique présent dans l'échantillon en CO₂, conversion par oxydation des composés azotés en nitrate, l'azote présent dans la liaison C-N étant transformé en ammonium,
- détermination du TOC à l'aide d'un détecteur IR,
- addition définie d'un réactif B dans le récipient d'attaque chauffé,
- irradiation de l'échantillon présent dans le récipient d'attaque avec la lumière UV,
- au cours d'une deuxième réaction d'attaque, conversion par oxydation de l'ammonium formé en nitrate,
- dégazage et ainsi transvasement dépourvu de bulles d'une partie de l'échantillon du récipient d'attaque à un détecteur de TN et
- mesure du TN,
- au cas où l'interrogation a pour résultat que le TOC et le TP doivent être déterminés (programme 140),
- insertion de l'échantillon liquide dans un récipient d'attaque chauffé,
- introduction d'azote ou d'air synthétique à titre de gaz support dans le récipient d'attaque chauffé,
- addition d'un réactif C dans le récipient d'attaque,
- irradiation de l'échantillon présent dans le récipient d'attaque avec la lumière UV,
- conversion du C organique en CO₂ et des composés de phosphore/phosphate en orthophosphate dans l'échantillon,
- détermination du TOC à l'aide d'un détecteur IR,
- dégazage et ainsi transvasement dépourvu de bulles d'une partie de l'échantillon du récipient d'attaque à un détecteur de TP,
- mesure du TP,
- au cas où l'interrogation a pour résultat que le TOC, le TN et le TP doivent être déterminés (programme 160),
- insertion de l'échantillon liquide dans un récipient d'attaque chauffé,
- introduction d'azote ou d'air synthétique à titre de gaz support dans le récipient d'attaque chauffé,
- addition d'un réactif C dans le récipient d'attaque,
- irradiation de l'échantillon présent dans le récipient d'attaque avec la lumière UV,
- au cours d'une première réaction d'attaque, conversion du C organique en CO₂ et des composés de phosphore/phosphate en orthophosphate, conversion des composés azotés en nitrate, l'azote présent dans la liaison C-N étant transformé en ammonium,
- détermination du TOC à l'aide d'un détecteur IR,
- dégazage et ainsi transvasement dépourvu de bulles d'une partie de l'échantillon du récipient d'attaque à un détecteur de TP,
- mesure du TP,
- addition définie d'un réactif B dans le récipient d'attaque chauffé,
- irradiation de l'échantillon présent dans le récipient d'attaque avec la lumière UV,
- au cours d'une deuxième réaction d'attaque, conversion par oxydation de l'ammonium formé en nitrate,
- dégazage et ainsi transvasement dépourvu de bulles d'une partie de l'échantillon du récipient d'attaque à un détecteur de TN,
- mesure du TN,
- au cas où l'interrogation a pour résultat que le TOC seulement doit être déterminé (programme 180),
- insertion de l'échantillon liquide dans un récipient d'attaque chauffé,
- introduction d'azote ou d'air synthétique à titre de gaz support dans le récipient d'attaque chauffé,
- addition d'un réactif A dans le récipient d'attaque,
- irradiation de l'échantillon présent dans le récipient d'attaque avec la lumière UV,
- conversion du C organique présent dans l'échantillon en CO₂, et
- détermination du TOC à l'aide d'un détecteur IR.

2. Procédé selon la revendication 1, **caractérisé en ce qu'**un nouveau cycle de l'interrogation en question est réalisé pour déterminer si une erreur de saisie en réponse à l'interrogation en question a été commise, au cas où aucune réponse positive n'a été donnée à l'interrogation en question demandant si le TN et/ou le TP devaient être déterminés en plus du TOC.

3. Procédé selon la revendication 1 ou 2, **caractérisé en ce que**, pour toutes les déterminations prévues, l'échantillon liquide est inséré une seule fois dans le récipient d'attaque et l'étape correspondante d'insertion de l'échantillon liquide dans le récipient d'attaque chauffé et d'introduction d'azote ou d'air synthétique à titre de gaz support n'est réalisée qu'une seule fois.

4. Procédé de détermination du TOC (carbone organique total) et de détermination du TN (azote total) dans un échantillon liquide, **caractérisé par** les étapes de procédé suivantes (programme 120) :
- insertion de l'échantillon liquide dans un récipient d'attaque chauffé,
- introduction d'azote ou d'air synthétique à titre de gaz support dans le récipient d'attaque chauffé,
- addition d'un réactif A dans le récipient d'attaque
- irradiation de l'échantillon présent dans le récipient d'attaque avec la lumière UV,
- au cours d'une première réaction d'attaque, conversion du C organique présent dans l'échantillon en CO₂, conversion des composés azotés en nitrate, l'azote présent dans la liaison C-N étant transformé en ammonium,
- détermination du TOC à l'aide d'un détecteur IR,
- addition définie d'un réactif B dans le récipient d'attaque chauffé,
- irradiation de l'échantillon présent dans le récipient d'attaque avec la lumière UV,
- au cours d'une deuxième réaction d'attaque, conversion par oxydation de l'ammonium formé en nitrate,
- dégazage et ainsi transvasement dépourvu de bulles d'une partie de l'échantillon du récipient d'attaque à un détecteur de TN et
- mesure du TN.

5. Procédé selon l'une des revendications 1 à 4, **caractérisé en ce que** le réactif A est une solution acide de peroxodisulfate (Na₂S₂O₈).

6. Procédé selon l'une des revendications 1 à 5, **caractérisé en ce que** le réactif B est une solution alcaline de peroxodisulfate.

7. Procédé selon l'une des revendications 1 à 3, 5 ou 6, **caractérisé en ce que** le réactif C est une solution de peroxodisulfate (Na₂S₂O₈) et de l'acide sulfurique (H₂SO₄).
